# EUROPEAN PATENT APPLICATION

(11) **EP 1 655 027 A1**
(43) Date of publication of application: **10.05.2006**
(21) Application number: 04026108.3
(22) Date of filing: 04.11.2004
(51) Int. Cl.: A61K 31/4535, A61P 1/00, A61P 9/08, A61P 9/00, A61P 15/00, A61P 25/06

(54) **9-(N-methyl-piperidyliden-4)-thioxanthene for treatment of pulmonary hypertension and migraine**

(71) Applicant: Biofrontera Pharmaceuticals GmbH, 51377 Leverkusen (DE)
(72) Inventor: Engels, Peter, Dr., 51465 Bergisch Gladbach (DE); Schmitz, Beate, Dr., 50825 Köln (DE)
(74) Representative: Sternagel, Fleischer, Godemeyer & Partner

(57) **Abstract**

The present invention relates to use of pimethixene as a medicament for the treatment of diseases aggravated by increased levels of 5-HT and mediated by 5-HT_{2B} receptors, such as for example pulmonary hypertension, migraine, disorders of the gastrointestinal tract, restenosis, prostatic hyperplasia and priapism.

## Description

The present invention relates to the use of 9-(N-METHYL-PIPERIDYLIDEN-4)-THIOXANTHENE as a medicament for the treatment of diseases aggravated by increased levels of 5-HT and mediated by 5HT_{2B} receptors like for example pulmonary hypertension or migraine.

Serotonin, a neurotransmitter with mixed and complex pharmacological characteristics, was first discovered in 1948, and subsequently has been the subject of substantial research. Serotonin, also referred to as 5-hydroxytryptamine (5-HT), acts both centrally and peripherally on discrete 5-HT receptors. Currently, fourteen subtypes of serotonin receptor are recognized and delineated into seven families, 5-HT₁, to 5-HT₇. Within the 5-HT₂ family, 5-HT_{2A}, 5-HT_{2B} and 5-HT_{2C} subtypes are known to exist. These subtypes share sequence homology and display similarities in their specificity for a wide range of ligands. Nomenclature and classification of 5-HT receptors have been reviewed (see Martin and Humphrey, Neuropharm. 1994, 33, 261-273 and Hoyer et al., Pharm. Rev. 1994, 46, 157-203).

The 5-HT_{2B} receptor, initially termed 5-HT_{2F}, or serotonin receptor like (SRL), was first characterized in rat isolated stomach fundus (see Clineschmidt et al., J. Pharmacol. Exp. Ther. 1985, 235, 696-708; Cohen and Wittenauer, J. Cardiovasc. Pharmacol. 1987, 10, 176-181) and initially cloned from rat (see Foguet et al., EMBO 1992, 11, 3481-3487) followed by the cloning of the human 5-HT_{2B} receptor (see Schmuck et al., FEBS Lett. 1994, 342, 85-90; Kursar et al., Mol. Pharmacol. 1994, 46, 227-234). The closely related 5-HT_{2C} receptor, widely distributed in the human brain, was first characterized as a 5-HT_{1C} subtype (see Pazos et al., Eur. J. Pharmacol. 1984, 106, 539-546) and was subsequently recognized as belonging to the 5-HT₂ receptor family (see Pritchett et al., EMBO J. 1988, 7, 4135-4.140).

Because of the similarities in the pharmacology of ligand interactions at 5-HT_{2B} and 5-HT_{2C} receptors, many of the therapeutic targets that have been proposed for 5-HT_{2C} receptor antagonists are also targets for 5-HT_{2B} receptor antagonists. Current evidence strongly supports a therapeutic role for 5-HT_{2B/2C} receptor antagonists in treating anxiety (e.g., generalized anxiety disorder, panic disorder and obsessive compulsive disorder), alcoholism and addiction to other drugs of abuse, depression, migraine, sleep disorders, feeding disorders (e.g., anorexia nervosa) and priapism. Additionally, current evidence strongly supports a therapeutic role for selective 5-HT_{2B} receptor antagonists that will offer distinct therapeutic advantages collectively in efficacy, rapidity of onset and absence of side effects. Such agents are expected to be useful in the treatment of hypertension, disorders of the gastrointestinal track (e.g., irritable bowel syndrome, hypertonic lower esophageal sphinter, motility disorders), restenosis, asthma and obstructive airway disease, and prostatic hyperplasia (e.g., benign prostatic hyperplasia).

EP 1 270 568 as well as WO 03/35646 describe selective 5HT_{2B} receptor antagonists which are derivatives of 4-(thio- or selenoxanthene-9-ylidene)-piperidine or acridine having in any case several side groups in the aromatic ring system.

The involvement of 5-HT receptor activation in the development of pulmonary hypertension has been described 1999 by M. MacLean in *TiPS,* Vol.20, p. 490-495. However, in this review article the disorder state is referred to activation of 5HT_{1D/1B} and 5HT_{2A} receptors.

J.-M. Launay et al. describe in *Nature Medicine* Vol. 8, Nr. 10 (2002), p. 1129-1135 that serotonin as well as other agonists of the 5-HT_{2B} receptor support the development of pulmonary hypertension. Further, the study showed that 5HT_{2B} receptor expression is upregulated in pulmonary hypertension.

The compound m-chlorophenylpiperazine (mCPP), known to trigger migraine-like head pain in some subjects, has the ability to induce dural plasma protein extravasation (PPE) in guinea pigs (Johnson et al. Cephalalgia 2003; 23/2 p.117). Intravenous mCPP dose-dependently increases PPE. This effect can be inhibited by non-selective 5-HT₂ receptor antagonists (methysergide, LY53857, LY215840), by a peripherally restricted 5-HT₂ receptor antagonist (xylamidine) and by a 5-HT_{2B} selective receptor antagonist (LY202146). These data suggest that peripheral 5-HT_{2B} receptors mediate mCPP-induced PPE. The nitric oxide synthase inhibitor L-NAME and the 5-HT₁ agonist sumatriptan also blocked mCPP-induced PPE, suggesting a role for nitric oxide (NO) and the trigeminal system, respectively. NO release has been linked to activation of 5-HT_{2B} receptors on the vascular endothelium. However, LY202146 did not block PPE induced by electrical stimulation of the trigeminal ganglion (Johnson et al. Cephalalgia 2003). These data are consistent with activation of peripheral 5-HT_{2B} receptors initiating PPE and the theory that selective 5-HT_{2B} antagonists might be effective prophylactic therapies for migraine. These data support the hypothesis that increased 5HT levels in the blood can induce PPE in blood vessels expressing 5-HT_{2B} receptors.

Pimethixene is the trivial name of 9-(N-methyl-piperidyliden-4)-thioxanthene. Pimethixene has been developed in the sixties and has been used for the treatment of sleep disorders, hyperactivity, anxiety, allergy, as bronchodilator and for anaesthesia.

It is the object of the present invention to provide a new medicament comprising a compound effective for treatment of disorders aggravated by increased levels of 5-HT in blood or blood vessels like pulmonary hypertension or migraine.

The object is met by use of a compound according to formula 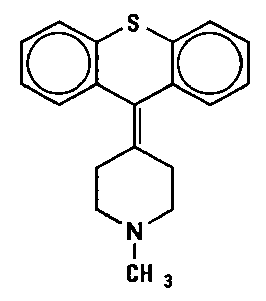 which compound is known as pimethixene or a pharmaceutically acceptable salt thereof for the development of a medicament for the treatment of disorders aggravated by increased levels of 5-HT like pulmonary hypertension or migraine.

The present invention further relates to a pharmaceutical composition comprising such a compound or a pharmaceutically acceptable salt thereof, in admixture with one or more pharmaceutically acceptable carriers.

"Pharmaceutically acceptable salt" refers to those salts which retain the biological effectiveness and properties of the free bases and which are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, ascorbic acid and the like.

The term "treatment" as used herein covers any treatment of a disease in a mammal, particularly a human, and includes:
(i) preventing the disease from occurring in a subject which may be predisposed to the disease but has not yet been diagnosed as having it;
(ii) inhibiting the disease, i.e., arresting its development; or
(iii) relieving the disease, i.e., causing regression of the disease.

The term "disease state " as used herein is intended to cover all disease states which are generally acknowledged in the art mediated by 5-HT₂ receptors due to to increased serotonin level and / or plasma protein extravasation from the blood into the surrounding tissue. Such disease states include, but are not limited to, migraine and pulmonary hypertension.

The compound of this invention has been found to be a human 5-HT_{2B} receptor antagonist. Affinity for the 5-HT_{2B} receptors was demonstrated using an in vitro binding assay utilizing cloned human 5-HT_{2B} receptors radiolabelled with [³H]-5HT, as well as luciferase reporter gene assays as shown in the examples. Antagonist properties for the human 5-HT_{2B} receptor was shown by counter screening at human 5-HT_{2A} and 5-HT_{2C} receptors. Affinity for the human H₁ receptor was determined using an in vitro binding assay utilizing cloned human H₁ receptors radiolabelled with [³H]-mepyramine and antagonistic properties were determined by an Luciferase reporter gene assay, as shown in the examples.

Accordingly, the compound of this invention is useful for treating diseases which can be ameliorated by blockade of 5-HT₂ and in particular 5HT_{2B} receptors. Because of the similarities in the pharmacology of ligand interactions at 5-HT_{2C} and 5-HT_{2B} receptors many of the therapeutic targets that have been proposed for 5-HT_{2C} receptor antagonists are also targets for 5-HT_{2B} receptor antagonists. In particular, several clinical observations suggest a therapeutic role for 5-HT_{2B} receptor antagonists in the prevention of migraine, in that mobilization of 5-HT into the plasma is believed to be a precipitating factor in migraine. Additionally, non-selective 5-HT_{2B} receptor agonists provoke migraine attacks in susceptible individuals, and non-selective 5-HT_{2B} receptor antagonists are effective in preventing the onset of migraine (see Kalkman, Life Sciences 1994, 54, 641-644). It is speculated that activation of 5-HT_{2B} receptors located on endothelial cells of meningeal blood vessels triggers migraine attacks through the formation of nitric oxide (see Schmuck et al., Eur. J. Neurosci. 1996, 8, 959-967).

Experimental evidence indicates that the compound of the present invention is useful in the treatment of migraine and pulmonary hypertension. 5-HT (serotonin) plays a key role in the regulation of transmission of nociceptive information at various levels of the peripheral and central nervous systems (See Richardson, B. P., "Serotonin and Pain", Ann. N.Y. Acad. Sci., 1990, 600, 511-520). Moreover, neuronal systems containing 5-HT are involved not only in the regulation of nociceptive input at the spinal and supraspinal level, but in mediating the nociceptive action of other analgesics including the opiates. 5-HT is a mediator of sensitization of nerve terminal nociceptors that may occur in the genesis of pain associated with inflammation. The 5-HT_{2B} receptor is highly sensitive to activation by 5-HT and specific blockade by selective 5-HT_{2B} antagonists may provide a novel avenue toward therapy.

Experimental evidence supports a therapeutic role for 5-HT_{2B} receptor antagonists in treating pulmonary hypertension. In pulmonary hypertension, one of the most profound increases in vascular responsiveness is observed for serotonin. Two lines of evidence imply that this results from a switch in the receptor mediating vasoconstriction from predominantly 5-HT_{2A} to predominantly 5-HT_{2B}. First, serotonin induced contractions of isolated blood vessels from hypertensive animals become resistant to block by selective 5-HT_{2A} receptor antagonists, but remain sensitive to non-selective 5-HT_{2B} receptor antagonists. Second, there is an increase in 5-HT_{2B} receptor mRNA in vessels from hypertensive animals (see Watts et al., J. Pharmacol. Exp. Ther. 1996, 277, 1103-13 and Watts et al., Hypertension 1995, 26, 1056-1059). This hypertension-induced shift in the population of receptor subtype mediating constrictor responses to 5-HT suggests that selective block of vasoconstrictor 5-HT_{2B} receptors may be of therapeutic benefit in the treatment of pulmonary hypertension.

In applying the compound of this invention to treatment of the above conditions, administration of the active compound and salts described herein can be via any of the accepted modes of administration, including inhalation, oral, parenteral and otherwise systemic route of administration. Any pharmaceutically acceptable mode of administration can be used, including solid, semi-solid or liquid dosage forms, such as, for example, tablets, suppositories, pills, capsules, powders, liquids, suspensions, aerosols or the like, preferably in unit dosage forms suitable for single administration of precise dosages, or in sustained or controlled release dosage forms for the prolonged administration of the compound at a predetermined rate. The compositions will typically include a conventional pharmaceutical carrier or excipient and at least the compound of the present invention or one of the pharmaceutically acceptable salts thereof and, in addition, may include other medicinal agents, pharmaceutical agents, carriers, adjuvants, etc..

The amount of the compound of the present invention administered will of course be dependent on the subject being treated, the severity of the affliction, the manner of administration and the judgment of the prescribing physician. However, an effective dose for inhalation, oral, parenteral and otherwise systemic routes of administration is in the range of 0.01-20 mg/kg/day, preferably 0.1-10 mg/kg/day. For an average 70 kg human, this would amount to 0.7-1400 mg per day, or preferably 7-700 mg/day.

One of ordinary skill in the art of treating such diseases will be able, without undue experimentation and in reliance upon personal knowledge and the disclosure of this application, to ascertain a therapeutically effective amount of the compound for a given disease.

For solid compositions, conventional non-toxic solid carriers include, for example, pharmaceutical grades of mannitol, lactose, cellulose, cellulose derivatives, sodium crosscarmellose, starch, magnesium stearate, sodium saccharin, talcum, glucose, sucrose, magnesium carbonate, and the like may be used. The active compound as defined above may be formulated as suppositories using, for example, polyalkylene glycols, e.g. PEG (polyethyleneglycol) or PEG derivatives, acetylated triglycerides and the like, as the carrier. Liquid pharmaceutically administrable compositions can, for example, be prepared by dissolving, dispersing, etc. an active compound as defined above and optional pharmaceutical adjuvants in a carrier, such as, for example, water, saline, aqueous dextrose, glycerol, ethanol, and the like, to thereby form a solution or suspension. If desired, the pharmaceutical composition to be administered may also contain minor amounts of nontoxic auxiliary substances such as wetting or emulsifying agents, pH buffering agents and the like, for example, sodium acetate, sorbitan monolaurate, triethanolamine sodium acetate, sorbitan monolaurate, triethanolamine oleate, etc. The composition or formulation to be administered will, in any event, contain a quantity of the active compound(s) in an amount effective to alleviate the symptoms of the subject being treated.

Pimethixene may be formulated in a suitable form for administration by inhalation (e.g. via an aerosol) or insufflation (either through the mouth or nose).

One manner for administering a compound of the invention is inhalation. Inhalation is an effective means for delivering an agent directly to the respiratory tract. There are three general types of pharmaceutical inhalation devices: nebulizer inhalers, dry powder inhalers (DPI), and metered-dose inhalers (MDI). Nebulizer devices produce a stream of high velocity air that causes a therapeutic agent to spray as a mist which is carried into the patient's respiratory tract. The therapeutic agent is formulated in a liquid form such as a solution or a suspension of micronized particles of respirable size, where micronized is typically defined as having about 90% or more of the particles with a diameter of less than about 10 .mu.m. A typical formulation for use in a conventional nebulizer device is an isotonic aqueous solution of a pharmaceutical salt of the active agent at a concentration of the active agent of between about 0.05 .mu.g/mL and about 10 mg/mL.

DPI's typically administer a therapeutic agent in the form of a free flowing powder that can be dispersed in a patient's air-stream during inspiration. In order to achieve a free flowing powder, the therapeutic agent can be formulated with a suitable excipient, such as lactose or starch. A dry powder formulation can be made, for example, by combining dry lactose having a particle size between about 1 .mu.m and about 100 .mu.m with micronized particles of a pharmaceutical salt of the active agent and dry blending. Alternative, the agent can be formulated without excipients. The formulation is loaded into a dry powder dispenser, or into inhalation cartridges or capsules for use with a dry powder delivery device.

Examples of DPI delivery devices provided commercially include Diskhaler (GlaxoSmithKline, Research Triangle Park, N.C.) (see, e.g., U.S. Pat. No. 5,035,237); Diskus (GlaxoSmithKline) (see, e.g., U.S. Pat. No. 6,378,519; Turbuhaler (AstraZeneca, Wilmington, Del.) (see, e.g., U.S. Pat. No. 4,524,769); and Rotahaler (GlaxoSmithKline) (see, e.g., U.S. Pat. No. 4,353,365). Further examples of suitable DPI devices are described in U.S. Pat. Nos. 5,415,162, 5,239,993, and 5,715,810 and references therein.

MDI's typically discharge a measured amount of therapeutic agent using compressed propellant gas. Formulations for MDI administration include a solution or suspension of active ingredient in a liquefied propellant. While chlorofluorocarbons, such as CCI.sub.3 F, conventionally have been used as propellants, due to concerns regarding adverse affects of such agents on the ozone layer, formulations using hydrofluoroalklanes (HFA), such as 1,1,1,2-tetrafluoroethane (HFA 134a) and 1,1,1,2,3,3,3,-heptafluoro-n-propane, (HFA 227) have been developed. Additional components of HFA formulations for MDI administration include co-solvents, such as ethanol or pentane, and surfactants, such as sorbitan trioleate, oleic acid, lecithin, and glycerin. (See, for example, U.S. Pat. No. 5,225,183, EP 0717987 A2, and WO 92/22286.)

Thus, a suitable formulation for MDI administration can include from about 0.01 % to about 5% by weight of a pharmaceutical salt of active ingredient, from about 0% to about 20% by weight ethanol, and from about 0% to about 5% by weight surfactant, with the remainder being the HFA propellant. In one approach, to prepare the formulation, chilled or pressurized hydrofluoroalkane is added to a vial containing the pharmaceutical salt of active compound, ethanol (if present) and the surfactant (if present). To prepare a suspension, the pharmaceutical salt can be provided as micronized particles. The formulation is loaded into an aerosol canister, which forms a portion of an MDI device. Examples of MDI devices developed specifically for use with HFA propellants are provided in U.S. Pat. Nos. 6,006,745 and 6,143,277.

In an alternative preparation, a suspension formulation is prepared by spray drying a coating of surfactant on micronized particles of a pharmaceutical salt of active compound. (See, for example, WO 99/53901 and WO 00/61108.) For additional examples of processes of preparing respirable particles, and formulations and devices suitable for inhalation dosing see U.S. Pat. Nos. 6,268,533, 5,983,956, 5,874,063, and 6,221,398, and WO 99/55319 and WO 00/30614.

With regard to construction of the delivery device, any form of aerosolization known in the art, including but not limited to nebulization, atomization or pump aerosolization of a liquid formulation, and aerosolization of a dry powder formulation, can be used in the practice of the invention. A delivery device that is uniquely designed for administration of solid formulations is envisioned. Often, the aerosolization of a liquid or a dry powder formulation will require a propellent. The propellent can be any propellent generally used in the art. Examples of useful propellents include chlorofluorocarbons, hydrofluorocarbons, hydrochlorofluorocarbons, and hydrocarbons, including trifluoromethanet dichlorodifluoroethane, dichlorotetrafluoroethanol, and 1,1,1,2-tetrafluoroethane, and combinations thereof.

Systems of aerosol delivery, such as the pressurized metered dose inhaler and the dry powder inhaler are disclosed in Newman, Aerosols and the Lung, Clarke, S. W. and Davia, D. editors, pp 197-22 and may be used in connection with the present invention.

Dosage forms or compositions containing pimethixene compound in the range of 0.25 to 95% by weight with the balance made up from non-toxic carrier may be prepared.

For oral administration, a pharmaceutically acceptable non-toxic composition is formed by the incorporation of any of the normally employed excipients, such as, for example pharmaceutical grades of mannitol, lactose, cellulose, cellulose derivatives, sodium crosscarmellose, starch, magnesium stearate, sodium saccharin, talcum, glucose, sucrose, magnesium, carbonate, and the like. Such compositions take the form of solutions, suspensions, tablets, pills, capsules, powders, sustained release formulations and the like. Such compositions may contain 1 to 95 % by weight of one of the compound of the present invention, more preferably 2 to 50 % by weight, most preferably 5 to 8 % by weight.

Parenteral administration is generally characterized by injection, either subcutaneously, intramuscularly or intravenously. Injectables can be prepared in conventional forms, either as liquid solutions or suspensions, solid forms suitable for solution or suspension in liquid prior to injection, or as emulsions. Suitable excipients are, for example, water, saline, dextrose, glycerol, ethanol or the like. In addition, if desired, the pharmaceutical composition to be administered may also contain minor amounts of non-toxic auxiliary substances such as wetting or emulsifying agents, pH buffering agents and the like, such as for example, sodium acetate, sorbitan monolaurate, triethanolamine oleate, triethanolamine sodium acetate, etc.

One manner for administering a compound of the invention is inhalation. Inhalation is an effective means for delivering an agent directly to the respiratory tract. There are three general types of pharmaceutical inhalation devices: nebulizer inhalers, dry powder inhalers (DPI), and metered-dose inhalers (MDI).

Transdermal or "pulsed" transdermal administration may be supported by cremes, gels, dispersions and the like.

A more recently devised approach for parenteral administration employs the implantation of a slow-release or sustained-release system, such that a constant level of dosage is maintained (see, e.g., US-A-3,710,795).

The percentage of active compound contained in such parental compositions is highly dependent on the specific nature thereof, as well as the activity of the compound and the needs of the subject. However, percentages of pimethixene of 0.1 to 10 % by weight in solution are employable, and will be higher if the composition is a solid which will be subsequently diluted to the above percentages. Preferably the composition will comprise 0.2 to 2 % by weight of pimethixene in solution.

The composition of the present invention may also be formulated for administration in any convenient way by analogy with other topical compositions adapted for use in mammals. These compositions may be presented for use in any conventional manner with the aid of any of a wide variety of pharmaceutical carriers or vehicles. For such topical administration, a pharmaceutically acceptable non-toxic formulation can take the form of semisolid, liquid, or solid, such as, for example, gels, creams, lotions, solutions, suspensions, ointments, powders, or the like. As an example, the active component may be formulated into a syrup or gel using ethanol, propylene glycol, propylene carbonate, polyethylene glycols, diisopropyl adipate, glycerol, water, etc., with appropriate gelling agents, such as Carbomers, Klucels, etc. If desired, the formulation may also contain minor amounts of non-toxic auxiliary substances such as preservatives, antioxidants, pH buffering agents, surface active agents, and the like. Actual methods of preparing such dosage forms are known, or will be apparent, to those skilled in the art; for example, see Remington's Pharmaceutical Sciences, Mack Publishing Company, Easton, Pa., 19th Edition, 1995.

Preferably the pharmaceutical composition is administered in a single unit dosage form for continuous treatment or in a single unit dosage form ad libitum when relief of symptoms is specifically required.

### Examples

### Example 1:

### Cloned Human 5-HT_{2B} Receptor Binding Assay

The following describes an in vitro binding assay utilizing cloned 5-HT_{2B} receptors radiolabelled with [³H]-5HT.

### Receptor Binding Assay

HEK 293 cells transiently transfected with an expression plasmid pXMD1-hu2B encoding the human 5-HT2B receptor (see Schmuck et al., FEBS Lett., 1994, 342, 85-90) were used as described previously (Schmuck et al., Eur. J. Pharmacol., 1996, 8, 959-967). Two days after transfection cells were harvested, pelleted at 500g for 5 min at 4°C, gently resuspended in ice-cold buffer1 (50 mM TRIS pH 7.7, 4 mM CaCl₂) and homogenized using a Polytron PT 1200 tissue homogenizer (position 6 for 30 s). Cells were pelleted at 50,000g, 4°C for 10 min, washed with buffer1 and pelleted again. The final pellet was resuspended in incubation buffer (50 mM TRIS pH 7.7, 4 mM CaCl₂, 10 µM pargyline and 0.1 % by weight ascorbic acid). The binding assay consisted of 300 µl of membrane suspension (protein concentration = 0.3 to 0.5 mg/ml), 150 µl of competing drug and 50 µl of [³H]5-HT at a final concentration of 4 to 5 nM. The mixture was incubated at 37°C for 30 min and the assay terminated by rapid filtration and two washing steps with 5 ml of cold 20 mM Tris-HCl pH = 7.5, and 154 mM NaCl over Whatman GFB filters. Filters were counted by liquid scintillation. Non-specific binding was determined in the presence of an excess of 5-HT (100 µM). Bound radioligand represented less than 1 % of free radioligand. In competition experiments, specific binding represented about 60 % of total binding. Results expressed as pKᵢ values are shown in Table 1

### Example 2:

### 5-HT_{2A} and 5-HT_{2C} Receptor Binding Methods

The following describes receptor binding methods in which the ligand with high affinity for 5-HT_{2B} receptors were counter screened at 5-HT_{2A} and 5-HT_{2C} receptors to demonstrate selectivity.

5-HT_{2A} receptors were labelled with [³H]ketanserin in human cortex, in HEK293 cells expressing a cloned human 5-HT_{2A} receptor and in HEK293 cells expressing the rat 5-HT_{2A} receptor. For competition binding studies the ligand concentration was approximately 0.1 nM. For saturation binding studies concentrations of radioligand ranged from 0.01 nM to 2.0 nM. Assays were conducted in 0.5 ml of assay buffer (50 mM Tris-HCl, 4 mM calcium chloride, 0.1 % by weight ascorbic acid) (pH 7.4 at 4°C). Non-specifc binding was defined with 10 mM unlabelled ketanserin. After a 60 min incubation at 32°C, membranes were harvested onto filters treated with 0.1 % by weight of polyethylenimine and the bound radioactivity was determined.

Human 5-HT_{2B} receptors were labelled in HEK293 cells as described above. except that the radioligand was [³H]-5HT and that the assay buffer contained pargyline in a concentration of 10 mM and 0.1 % by weight of ascorbic acid. For competition binding studies the radioligand concentration was approximately 0.4 nM while for saturation binding studies the concentration of [³H]-5HT ranged from 0.05 to 8 nM. Non-specific binding was defined with 10 mM 5-HT. Incubations were for 120 min at 4°C.

5-HT_{2C} receptors were labelled in Cos-7 cells expressing the human 5-HT_{2C} receptor and in NIH-3T3 expressing the rat 5-HT_{2C} receptor.

Assays were conducted as described for the 5-HT_{2A} receptor except that the radioligand was [³H]mesulergine. The radioligand concentration for competition studies was approximately 0.2 nM while for saturation binding studies the concentration ranged from 0.1 to 18 nM. Non-specific binding was defined with 10 µM unlabelled mesulergine.

Competition radioligand binding data were analyzed using a four parameter logistic equation and iterative curve-fitting techniques to obtain estimates of the IC₅₀ and Hill slope. Kd values, determined from saturation binding studies were then used to calculate inhibition dissociation constants (Ki).

Proceeding as in the example above the compound of the present invention was found to have selective affinity for the 5-HT_{2B} receptor versus 5HT_{2C} receptor. Results are shown in Table 1

### Example 3:

### Cloned Human H₁, H₂, and H₃ Receptor Binding Assay

CHO cells were stably transfected with an expression plasmid pCineohH1, pCineohH2, pCineohH3 and pCineohH4 encoding the human Histamine H₁, H₂, H₃ or H₄ receptor, respectively. Recombinant cell lines were harvested after confluent growth, homogenized in ice-cold 50 mM Na₂/potassium phosphate buffer (pH 7.4) and used for radioligand binding studies. Cell homogenates (40 - 50 µg of protein) were incubated for 30 min at 25°C in 50 mM Na₂/potassium phosphate buffer (pH 7.4) in 400 µl with the various concentrations of either [³H]-mepyramine, [³H]-thiotidine, [³H]-R-α-Methylhistamine, and [³H]-pyramilamine for cells expressing recombinant human H₁, H₂, H₃ and H₄ receptors, respectively. The nonspecific binding was defined in the presence of 1 µM mianserin. In displacement studies, cell homogenated were incubated either with 1 nM [³H]-mepyramine, 15 nM [³H]-thiotidine, 0.5 nM [³H]-R-α-Methylhistamine, or 15 nM [³H]-pyramilamine and increasing concentrations of competing ligands. The incubations were stopped by rapid dilution with 3 ml of ice-cold 50 mM Na₂/potassium phosphate buffer (pH 7.4). The bound radioactivity was seperated by filatration through Whatman GF/C filters that had been treated with 0.3 % polyethyleneimine. Filters were washed twice in 3 ml of buffer and radioactivity retained on the filters was measured by liquid scinitllation counting.
The concentration of Pimethixene producing 50% inhibition of binding (IC₅₀) was determined using iterative curve fitting techniques.

Proceeding as in the example above, the compound of the present invention was found to have no affinity for the human H_{2,3} and H4 receptor while it showed high affinity to the H 1 receptor.

**Table 1: Receptor Affinity: pK Values of the Pimethixene**

| Receptor | **Pimethixene** | Methysergide | Pizotifen |
|---|---|---|---|
| | pKi values | | |
| 5HT_{2A} | **10.2** | 8,54 | |
| 5HT_{2B} | **10.6** | 8,85 | 8,54 |
| 5HT_{2C} | **8.6** | | |
| H₁ | **11.1** | | |
| H₂ | low activity | | |
| H₃ | low activity | | |
| H₄ | low activity | | |
| *M*_{*1*} | 8.6 | | |
| *D*_{*2*} | 8.2 | | |
| *Alpha* _{*1A*} | 7.6 | | |

Summary of pKi values of Pimethixene for the respective receptor subtypes. pKi values were calculated using the equation of Cheng and Prusoff {pKi = -log (IC₅₀/[1+ used concentration of Agonist/EC₅₀])}.EC₈₀ values were calculated from corresponding dose-response experiment using 5-HT, histamine, epinephrine (alpha 1A adrenergic receptor), dopamine (D2 dopaminergic receptor), and acetylcholine (M1 muscarinic receptor) as agonists.

The results of table 1 show, that the compound of the present invention has a selective affinity for the 5HT_{2A/B} and H₁ receptor, particularly have a much higher affinity for the 5HT_{2B} receptor than for the 5HT_{2C} receptor or other monoaminergic receptors.

### Example 4:

Functional test of Pimethixene in the mCPP model in guinea pigs.

In accordance to the protocol published by Markowitz et. al; (1987) and Olesen et. al; (2000), guinea pigs were used to determine the amount of plasma protein released at the blood vessels within the Dura mater after mCPP application.
After deep anesthesia the test compounds pimethixene, methysergide or pizotifen were injected intravenous in the Vena jugularis at different concentrations. 2 minutes later 1µg/kg body weight mCPP were injected and followed by the i.v. application of EvansBlue as marker for the protein plasma extravasation. 15 minutes later animals were transcranial perfused and decapitated. A,defined area of the dura mater was isolated and transferred into an eppendorf tube containing 200µl formamid. Because EvansBlue binds to plasma protein, the amount of bound marker dye is a measure of extravasation of plasma protein. Formamid dissolves EvansBlue from the protein which can be colorimetric detected and quantified at 600 nm.

**Table 2: Determination of plasma protein extravasation as EvansBlue extinction at 600nm.**

| Dosis in µg/kg | **Pimethixene** | **n** | Pizotifen | n | Methysergide | n |
|---|---|---|---|---|---|---|
| 100 | **not determined** | **-** | 0,230 ± 0,018 | 7 | 0,189 ± 0,026 | 3 |
| 10 | **not determined** | **-** | 0,144 ± 0,035 | 8 | 0,228 ± 0,064 | 5 |
| 1 | **not determined -** | | 0,404 ± 0,025 | 6 | 0,250 ± 0,027 | 5 |
| 0,1 | **0,126 ± 0,028** | **5** | not determined - | | 0,265 ± 0,034 | 5 |
| 0,01 | **0,157 ± 0,040** | **4** | not determined - | | not determined | - |
| 0,001 | **0,269 ± 0,022** | **5** | not determined - | | not determined | - |

An extinction of 0,300 ± 0,038 was determined for the positive control (mCPP agonist), an extinction of 0,130 ± 0,024 was measured for the negative control.

As shown in table 2, pimethixene inhibited the plasma protein extravasation at concentrations 100 to 1000fold lower than the reference compounds methysergide or pizotifen.

## Claims

1. Use of a compound according to the Formula 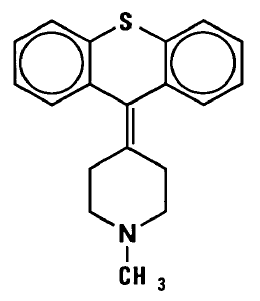 or a pharmaceutically acceptable salt thereof for the preparation of a medicament for the treatment of diseases aggravated by increased levels of 5-HT and mediated by 5-HT_{2B} receptors.

2. The use of claim 1 wherein the disease state is selected from migraine, pulmonary hypertension, disorders of the gastrointestinal tract, restenosis, prostatic hyperplasia and priapism.

3. The use of claim 1 wherein the disease state is migraine or pulmonary hypertension.

4. A pharmaceutical composition comprising a compound according to claim 1 or a pharmaceutically acceptable salt thereof, in admixture with one or more pharmaceutically acceptable carriers for the treatment of pulmonary hypertension.

5. A pharmaceutical composition comprising a compound according to claim 1 or a pharmaceutically acceptable salt thereof, in admixture with one or more pharmaceutically acceptable carriers for the treatment of migraine.
